# EUROPÄISCHE PATENTANMELDUNG

(11) **EP 0 947 832 A1**
(43) Veröffentlichungstag der Anmeldung: **06.10.1999**
(21) Anmeldenummer: 99106302.5
(22) Anmeldetag: 26.03.1999
(51) Int. Cl.: G01N 33/574, G01N 33/531

(54) **Immunologisches Verfahren zur Bestimmung von PSA**

(30) Priorität: 03.04.1998 DE 19814915
(71) Anmelder: Roche Diagnostics GmbH, 68298 Mannheim (DE)
(72) Erfinder: Hösel, Wolfgang Dr., 82327 Tutzing (DE); Peter, Jochen Dr. Nat. Inst. Environmental Health, Res. Triangle Park, North Carolina 27709 (US); Unverzagt, Carlo Prof. Dr., 81371 München (DE)

(57) **Zusammenfassung**

Die Erfindung betrifft ein immunologisches Verfahren zur Bestimmung von Prostataspezifischem Antigen (PSA), insbesondere zur Bestimmung von freiem PSA, dem Gesamtgehalt an PSA und dem Gehalt an PSA-Serpin-Komplexen durch Inkubation der Probe mit mindestens einem Antikörper, der spezifisch an freies PSA, aber nicht an komplexiertes PSA bindet. Das Verfahren ist dadurch gekennzeichnet, daß die Probe insbesondere vor Bestimmung des Gesamtgehalts an PSA mit einem aminhaltigen Nukleophil versetzt wird. Weiterhin betrifft die Erfindung die Verwendung von aminhaltigen Nukleophilen zur Herstellung von freiem PSA sowie ein Verfahren zur Herstellung von freiem PSA aus komplexiertem PSA durch Inkubation einer Probe, die komplexiertes PSA enthält, mit einem aminhaltigen Nukleophil.

## Beschreibung

Gegenstand der vorliegenden Erfindung ist ein immunologisches Verfahren zur Bestimmung von Prostata-spezifischem Antigen (PSA), insbesondere zur Bestimmung von freiem PSA, dem Gesamtgehalt an PSA und dem Gehalt an PSA-Serpin-Komplexen durch Inkubation der Probe mit mindestens einem Antikörper, der spezifisch an freies PSA, aber nicht an komplexiertes PSA bindet. Das Verfahren ist dadurch gekennzeichnet, daß die Probe vor Bestimmung des Gesamtgehalts an PSA mit einem aminhaltigen Nukleophil versetzt wird. Weiterhin betrifft die Erfindung die Verwendung von aminhaltigen Nukleophilen zur Herstellung von freiem PSA sowie ein Verfahren zur Herstellung von freiem PSA aus komplexiertem PSA durch Inkubation einer Probe, die komplexiertes PSA enthält, mit einem aminhaltigen Nukleophil.

Das Prostata-spezifische Antigen ist ein Glykoprotein mit einem Molekulargewicht von 29 kDa. Es wird in den Prostata-Epithelzellen gebildet und ist ein Bestandteil der Samenflüssigkeit. PSA hat die enzymatische Aktivität einer neutralen Serinprotease.

Die Hauptfunktion des PSA besteht in der Spaltung der Seminogeline I und II sowie des Fibronektins, die als wesentliche Komponenten des Ejakulats die Beweglichkeit der Spermien blockieren. Durch die Hydrolyse dieser Proteine bewirkt PSA die Verflüssigung des Samenkoagulums und ermöglicht so die Mobilität der Spermien.

Das enzymatisch aktive PSA wird im Serum durch verschiedene Inhibitoren inaktiviert. Zu den wichtigsten Inhibitoren gehören die sogenannten Serpine (= Serinprotease-Inhibitoren), die das PSA durch Bildung von kovalenten Komplexen inaktivieren. Die Hauptmenge des immunologisch nachweisbaren PSA ist im Serum an α1 -Antichymotrypsin (= ACT), das zu den Serpinen gehört, gebunden (zu 60-95 %). Weitere Komplexe können mit α1 -Antitrypsin und Protein C-Inhibitor gebildet werden, die aber im Vergleich zu PSA-ACT nur in sehr untergeordnetem Maße im Serum vorhanden sind.

Darüberhinaus bildet PSA auch noch mit einem anderen Typ von Protease-Inhibitor. nämlich dem α2-Makroglobulin (α2-M) einen Komplex, über dessen Gehalt im Serum in der Literatur unterschiedliche Angaben gemacht werden, vor allem da das PSA in diesem Komplex immunologisch nicht zugänglich ist. Daneben kommt im Serum noch enzymatisch inaktives freies PSA vor, das keine Komplexe eingehen kann. Unter der Bezeichnung Gesamt-PSA ist nachfolgend die Summe von freiem und mit Serpinen komplexiertem PSA gemeint, da der PSA-Komplex mit α2-M in allen bisherigen immunologischen Bestimmungen nicht erfaßt wird (Tewari und Bluestein, J. Clin. Ligand Assay 1995, Vol. 18, S. 186-196).

α1-Antichymotrypsin ist ein Glykoprotein mit einem Molekulargewicht von ca. 60 kDa. Als einer der Hauptinhibitoren in der akuten Phase spielt ACT eine wichtige Rolle bei der Kontrolle von Entzündungen. ACT bildet auch Komplexe mit Chymotrypsin Cathepsin G und glandulärem Kallikrein hK2. ACT kommt im Humanserum bezogen auf die molaren Verhältnisse in 10.000-fach höherer Konzentration vor als PSA.

Aufgrund des Vorkommens verschiedener PSA-Formen liegen für die Bestimmung der PSA-Konzentration in humanen Seren zur Diagnose eines Prostata-Carcinoms oder einer benignen Erkrankung sehr komplexe Verhältnisse vor. Diese Tatsache führt zu einer Reihe von diagnostischen Schwierigkeiten und schränkt den Wert dieses Markers in gewissem Maße ein. So ist bekannt, daß einige der verfügbaren Tests zur Bestimmung von PSA aufgrund der Spezifität der eingesetzten Antikörper unterschiedliche Werte für freies und komplexiertes PSA liefern (Zhou et al.. Clin. Chem. 1993, Vol 39/12. S. 2483-2491; McCormack et al., Urology 1995, Vol. 45, S. 729-744; Tewari und Williams. Clin. Chem. 1998, Vol. 44, S. 191-192; Blase und Sokoloff, Clin. Chem. 1998. Vol. 44. S. 192-193). Darüberhinaus ist aufgrund des Vorliegens der verschiedenen Formen eine genaue und einheitliche Standardisierung schwierig (Chen et al., Clin. Chem. 1995, Vol. 41, S. 1273-1282).

Besonders problematisch ist die Aussagekraft der PSA-Werte im Serum als Indikator für ein Prostata-Carcinom (PCa) bis zu einer Konzentration von ca. 15 ng/ml, da solche Mengen an gesamtem PSA auch durch das Vorliegen einer benignen Prostatahyperplasie (BPH) begründet sein können. Ein Screening auf Prostata-Carcinom ist aufgrund der mangelnden Spezifität in diesem Konzentrationsbereich durch Bestimmung des PSA-Wertes im Serum allein nicht möglich, sondern es müssen weitere, zum Teil aufwendige und schmerzhafte Untersuchungen (Biopsie) zur Abklärung des möglichen Vorliegens eines PCa herangezogen werden.

In den letzten Jahren hat die Bestimmung des Verhältnisses von freiem, das heißt unkomplexiert vorliegenden PSA, zu komplexiertem bzw. dem Gesamtgehalt an PSA eine gewisse Verbesserung der Spezifität gebracht (siehe zum Beispiel WO 92/01936). Allerdings liegt diese immer noch sehr deutlich unter derjenigen, die notwendig wäre, um eine größere Zahl an unnötigen und aufwendigen Folgeuntersuchungen zu vermeiden. Basis für die Verbesserung der Spezifität ist die Tatsache, daß in Seren von Patienten mit BPH das Verhältnis von freiem zu komplexiertem PSA (PSA 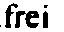 /PSA 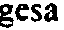 ) im Durchschnitt höher ist als bei Seren von PCa-Patienten.

Im Stand der Technik müssen bisher für die Ermittlung des Quotienten PSA 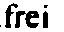 /PSA 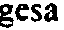 zwei unterschiedliche Messungen durchgeführt werden. Zunächst muß die Konzentration des freien PSA bestimmt werden. Wird dieser Test auf freies PSA im Sandwichformat durchgeführt, so muß mindestens einer der beiden Antikörper spezifisch für freies PSA sein und darf komplexiertes PSA nicht binden. Parallel dazu muß in einer zweiten separaten Messung die Konzentration des gesamten PSA ermittelt werden. In diesem Fall werden im Sandwichtest Antikörper verwendet, die freies und komplexiertes PSA binden. Alternativ kann die Ermittlung der Gesamtkonzentration auch durch Messung der PSA-ACT-Konzentration erfolgen. In der WO 92/01936 wird zum Nachweis der PSA-ACT-Konzentration ein solcher Antikörper, der freies und komplexiertes PSA bindet, sowie ein Antikörper, der spezifisch nur ACT bindet, verwendet. Die Gesamtmenge an PSA erhält man dann durch Addieren dieses Meßwerts für den PSA-ACT-Komplex und dem Meßwert für freies PSA. Ein großer Nachteil bei den Verfahren des Standes der Technik ist es, daß zwei verschiedene Tests durchgeführt werden, bei denen jeweils mindestens zwei Antikörper mit verschiedenen Spezifitäten eingesetzt werden müssen. Die Verwendung verschiedener Reagenzien, z.B. Antikörper, in mehreren unterschiedlichen Testführungen zur Bestimmung eines diagnostisch relevanten Parameters führt mit hoher Wahrscheinlichkeit zu Problemen in der Vergleichbarkeit von Tests unterschiedlicher Hersteller, vor allem bei einer solch komplexen diagnostischen Fragestellung wie bei PSA. Darüberhinaus erhöht sich mit dem Anstieg der zu verwendenden Testsysteme sowie der dabei notwendigen Reagenzien auch die Wahrscheinlichkeit von Störungen dieser Bestimmungen. Weiterhin erfordert die Bereitstellung vieler verschiedener, im allgemeinen monoklonaler Antikörper, einen erheblichen Entwicklungs- und Kostenaufwand.

Aufgabe war es daher, ein verbessertes und vereinfachtes immunologisches Verfahren zum Nachweis von PSA, insbesondere zum Nachweis von freiem PSA, dem Gesamtgehalt an PSA und dem Gehalt an PSA-Serpin-Komplexen zu entwickeln, das die Nachteile des Standes der Technik weitgehend überwindet.

Die Aufgabe wird gelöst durch ein verbessertes immunologisches Verfahren zur quantitativen Bestimmung von PSA durch Inkubation der Probe mit mindestens einem Antikörper, der spezifisch an freies PSA, aber nicht an komplexiertes PSA bindet, das dadurch gekennzeichnet ist, daß vor der Bestimmung des Gesamtgehalts an PSA die Probe mit einem aminhaltigen Nukleophil versetzt wird. Bevorzugt wird das Verfahren zur quantitativen Bestimmung von freiem PSA, dem Gesamtgehalt an PSA und dem Gehalt an PSA-Serpin-Komplexen angewandt.

Der Komplex aus PSA und Serpinen, insbesondere ACT, ist stabil und widersteht auch recht drastischen Bedingungen bezüglich Temperatur und pH-Werten (McCormack et al., Urology 1995, Vol. 45, S. 729-744). Überraschenderweise hat es sich nun gezeigt, daß der PSA-Serpin-Komplex durch Zugabe von aminhaltigen nukleophilen Reagentien gespalten werden kann. Anhand von HPLC-Analysen hat sich herausgestellt, daß das dabei entstehende freie PSA stabil ist. Überraschenderweise ist dies auch in Anwesenheit von Serum der Fall, das heißt, es erfolgt keine erneute Komplexierung. Dies ist daran zu erkennen, daß der Wert des freien PSA nach Inkubation unter Spaltungsbedingungen dem Wert des gesamten PSA vor der Spaltung entspricht. Überraschend ist dieser Befund deshalb, weil freies PSA im Serum aufgrund des vorliegenden jeweils sehr hohen Überschusses der Proteaseinhibitoren α1-Antichymotrypsin und α2-Makroglobulin eigentlich wieder komplexiert werden müßte. Dies ist zumindest dann der Fall, wenn zu einer Humanserum-Probe zusätzlich PSA zugesetzt wird (Malm und Lilja, Scand. J. Clin. Invest. 1995 Vol. 55. S. 15-22; Huber et al. The Prostate 1995, Vol 27, S. 166-175).

Durch die damit praktisch nicht reversible Spaltung des PSA-Serpin-Komplexes (insbesondere des PSA-ACT-Komplexes) ergibt sich daher die Möglichkeit, zunächst die Konzentration an freiem PSA in der unbehandelten Probe zu ermitteln. Dies geschieht bevorzugt mittels eines Immunoassays im Sandwichformat, bei dem zumindest einer der Antikörper spezifisch freies PSA, aber nicht komplexiertes PSA bindet. Zu einer identischen Parallelprobe wird ein aminhaltiges nukleophiles Reagenz gegeben, wodurch das mit Serpinen komplexierte PSA (vor allem PSA-ACT) gespalten wird und das zuvor komplexierte PSA nun in freier Form vorliegt. Anschließend wird im gleichen Meßverfahren, das heißt mittels der gleichen Antikörper, die Konzentration an freiem PSA bestimmt. Der hier ermittelte Wert enspricht der Gesamtkonzentration an PSA. Unter den gleichbedeutenden Begriffen 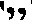 Gesamtkonzentration an PSA", 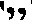 GesamtPSA", 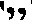 Gesamtgehalt an PSA" und 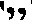 PSA_{gesamt} "wird analog zum Sprachgebrauch im Stand der Technik die Menge an freiem PSA und an PSA, das mit Serpinen komplexiert ist, verstanden.

Durch Division der Konzentration für freies PSA durch diejenige für Gesamt-PSA ergibt sich der aussagekräftige Quotient PSA _{frei}/PSA_{gesamt}. Darüberhinaus kann durch Subtraktion des Wertes für PSA_{frei} vom PSA_{gesamt}-Wert auch die Konzentration an mit Serpinen komplexiertem PSA ermittelt und anschließend der Quotient PSA_{frei}/PSA-Serpin-Komplex bzw. PSA_{frei}/PSA-ACT bestimmt werden. Anhand dieser Quotienten kann mit einer gewissen Wahrscheinlichkeit auf das Vorliegen einer BPH oder eines PCa geschlossen werden. Je größer der Quotient ist, desto wahrscheinlicher ist das Vorliegen einer benignen Erkrankung.

Zweckmäßig ist die Durchführung des erfindungsgemäßen Verfahren zur Bestimmung von freiem PSA und dem Gesamtgehalt an PSA mit folgenden Schritten: a) Inkubation eines Teils der Probe mit mindestens einem Antikörper, der spezifisch an freies PSA, aber nicht an komplexiertes PSA bindet. b) Bestimmung der Konzentration an freiem PSA, c) Inkubation eines Teils der Probe mit einem aminhaltigen nukleophilen Reagenz, d) Inkubation der Probe aus Schritt c) mit einem Antikörper gemäß Schritt a), e) Bestimmung des Gesamtgehalts an PSA der Probe gemäß Schritt b), und f) Ermittlung des Verhältnisses an freiem PSA zum Gesamtgehalt an PSA anhand der Werte aus Schritt b) und Schritt e).

Soll der Quotient PSA_{frei}/PSA-Serpin-Komplex bzw. PSA_{frei}/PSA-ACT ermittelt werden, so wird als Schritt f) vom Gesamtgehalt an PSA die Konzentration an freiem PSA aus Schritt b) subtrahiert und als Schritt g) der Quotient aus freiem PSA gemäß Schritt b) und dem PSA-Serpin-Komplex-Wert bzw. PSA_{frei}/PSA-ACT-Wert aus Schritt f) gebildet.

Wesentlich ist für die erfolgreiche Durchführung des Verfahrens, daß das durch die erfindungsgemäße Spaltung erhaltene freie PSA immunologisch reaktiv ist. Dies bedeutet, daß die Epitope, die von den Antikörpern erkannt werden, auch nach der Spaltung durch aminhaltige nukleophile Reagenzien vollständig erhalten bleiben. Ein wesentlicher Vorteil des erfindungsgemäßen Verfahrens ist es, daß für die Bestimmung von freiem und von gesamtem PSA jeweils die gleichen Antikörper, das heißt Antikörper gleicher Spezifität verwendet werden können. Die aufwendige Entwicklung von Antikörpern verschiedener Spezifitäten, wie sie nach den Verfahren des Standes der Technik erforderlich sind, wird dadurch vermieden. Je mehr verschiedene Antikörper verwendet werden, desto größer ist die Gefahr von unspezifischen Wechselwirkungen mit Probenbestandteilen. Da im erfindungsgemäßen Verfahren zumeist nur zwei verschiedene Antikörper verwendet werden, verringern sich diese Störeinflüsse deutlich gegenüber dem Stand der Technik.

Als Testformate für das erfindungsgemäße Verfahren sind heterogene und homogene Testführungen geeignet. Als besonders vorteilhaft hat sich der heterogene klassische Sandwichassay erwiesen. Hierbei wird der Analyt (hier das Antigen PSA bzw. in den Verfahren des Standes der Technik auch der PSA-ACT-Komplex) zwischen einem festphasengebundenen und einem markierten Bindepartner (hier: Antikörper) sandwichartig gebunden. Der Nachweis der Markierung erfolgt in dem Fachmann bekannter Weise. Übliche Markierungen sind Enzyme, zur Lumineszenz fähige Substanzen, Haptene wie Digoxigenin, fluoreszierende und radioaktive Substanzen. Als Festphase können beispielsweise Kunststoffröhrchen, Mikrotiterplatten, Latexpartikel oder Magnetbeads verwendet werden. Bevorzugt wird eine mit Streptavidin oder Avidin beschichtete Oberfläche verwendet. Die Bindung des Antikörpers an die Festphase kann direkt über Adsorption oder bevorzugt indirekt erfolgen. Die indirekte Bindung erfolgt bevorzugt über die Bindung eines mit Biotin gekoppelten Antikörpers an die mit Streptavidin oder Avidin beschichtete Festphase.

Denkbar ist auch der Nachweis von PSA mittels kompetitiver Testführung. Hierbei wird ein festphasengebundener Komplex aus zwei füreinander spezifischen Bindepartnern gebildet, wobei der Bindepartner, der nicht direkt an die Festphase gekoppelt ist, markiert ist. Der Analyt, je nach Testanforderung ein Antigen oder ein Antikörper, verdrängt entsprechend seiner Konzentration den markierten Bindepartner aus dem Komplex. Nach Trennung der festen von der flüssigen Phase wird die Markierung in einer der Phasen nachgewiesen. Für einen PSA-Test mit kompetitiver Testfürung wäre beispielsweise der festphasengebundene Bindepartner ein Antikörper, der spezifisch für freies PSA ist. Der andere Bindepartner wäre dann markiertes freies PSA. Dieses markierte freie PSA wird dann durch das freie PSA in der Probe in Abhängigkeit von dessen Konzentration verdrängt.

Die hier exemplarisch genannten Testformate und ihre Durchführung sowie der Nachweis der Analyten sind hier nur beispielhaft aufgeführt und dienen lediglich der Veranschaulichung. Die Verfahren sind dem Fachmann geläufig und bedürfen hier keiner weiteren Erläuterung.

In allen Testformaten muß mindestens ein Antikörper, der spezifisch an freies, nicht aber an komplexiertes PSA bindet, eingesetzt werden. Dies kann ein monoklonaler oder polyklonaler Antikörper sein. Bevorzugt wird jedoch ein monoklonaler Antikörper eingesetzt. Es können sowohl ganze Antikörper als auch Fragmente wie Fab, F(ab)', F(ab)'₂ eingesetzt werden. Wesentlich ist die Spezifität für freies PSA. Das heißt, dieser für freies PSA spezifische Antikörper darf nur mit unkomplexiertem PSA reagieren. Kreuzreaktivitäten mit komplexiertem PSA dürfen nicht oder nur vernachlässigbar schwach vorliegen, so daß sie den Test nicht verfälschen. Sollten weitere Antikörper notwendig sein, so können diese ebenfalls als intakte mono- oder polyklonale Antikörper oder als Fragmente davon eingesetzt werden. Die Anforderungen für die weiteren Antikörper sind dergestalt, daß sie freies PSA erkennen müssen. Eine Kreuzreaktion mit komplexiertem PSA ist bei den weiteren Antikörpern jedoch nicht kritisch.

Als Proben können erfindungsgemäß alle dem Fachmann geläufigen biologischen Flüssigkeiten verwendet werden. Bevorzugt werden als Probe Körperflüssigkeiten wie Vollblut, Blutserum, Blutplasma, Sperma oder Urin eingesetzt.

Erfindungsgemäß werden zur Spaltung der PSA-Serpinkomplexe (insbesondere von PSA-ACT) nukleophile aminhaltige Reagenzien eingesetzt. Dazu gehören bevorzugt Aminoalkohole wie beispielsweise Ethanolamin und Diethanolamin. Besonders bevorzugt wird Ethanolamin verwendet. Die PSA-ACT-Komplexe werden gespalten, indem zu einer beliebigen Probe, die PSA in komplexierter Form enthält, das nukleophile aminhaltige Reagenz dazugegeben wird. Als geeignet hat sich eine Konzentration des nukleophilen aminhaltigen Reagenz von 0,05 bis 2 M erwiesen. Bevorzugt wird es in einer Konzentration von 0.1 bis 1 M eingesetzt. Der pH-Wert in der Probe liegt bevorzugt zwischen 9 und 11. Die Inkubationstemperatur der so behandelten Probe mit dem nukleophilen aminhaltigen Reagenz ist unkritisch. Als geeignet hat sich eine Temperatur zwischen 10 und 40 °C, bevorzugt zwischen 20 und 30 °C, besonders bevorzugt von 25 °C erwiesen. Die Inkubation findet im allgemeinen über mehrere Stunden statt. Da das durch Spaltung entstehende freie PSA stabil ist,. sind Inkubationszeiten von 24 Stunden und länger für das erfindungsgemäße Verfahren möglich. Gegenstand der vorliegenden Erfindung ist daher auch ein Verfahren zur Herstellung von freiem PSA durch Inkubation einer Probe, die komplexiertes PSA enthält, mit einem nukleophilen aminhaltigen Reagenz unter den zuvor genannten Bedingungen. Das so hergestellte freie PSA kann dann beispielsweise für weitere diagnostische oder wissenschaftliche Struktur- oder Funktionsuntersuchungen eingesetzt werden.

Ein weiterer Gegenstand der Erfindung ist daher die Verwendung des entsprechend dem erfindungsgemäßen Verfahren hergestellten freien PSA für nicht-immunologische Untersuchungen wie beispielsweise Massenspektrometrie.

Ebenfalls ein Gegenstand der Erfindung ist die Verwendung eines nukleophilen aminhaltigen Reagenz, bevorzugt eines Aminoalkohols und besonders bevorzugt die Verwendung von Ethanolamin zur Herstellung von freiem PSA.

Das gemäß dem obigen Verfahren hergestellte PSA kann erfindungsgemäß auch als Standard verwendet werden. Ein solcher Standard kann zur Erstellung von Eichkurven in Immunoassays zum quantitativen Nachweis von PSA eingesetzt werden.

Ein weiterer Gegenstand der Erfindung ist die Verwendung eines nukleophilen, aminhaltigen Reagenzes in einem immunologischen Verfahren zum Nachweis von PSA. Bevorzugt wird Ethanolamin eingesetzt.

Ein weiterer Gegenstand der Erfindung ist ein Reagenz, enthaltend mindestens ein nukleophiles aminhaltiges Reagenz, bevorzugt Ethanolamin, zur Verwendung in einem Immunoassay zum Nachweis von PSA, sowie weitere dem Fachmann geläufige Bestandteile wie Puffer, Salze und Detergenzien. Das Reagenz wird bevorzugt in flüssiger, wässriger Form bereitgestellt.

Die folgenden Beispiele erläutern die Erfindung weiter.

### Beispiel 1: Spaltung von PSA-ACT mit aminhaltigen Nukleophilen

Als Proben werden kommerziell erhältliche Substanzen der Scripps Laboratories, San Diego, Californien, USA, eingesetzt: PSA_{frei}(lyophilisiert) Katalognr. P0714, PSA-ACT Katalognr. P0624. Beide Lyophilisate werden jeweils mit bidestilliertem Wasser resuspendiert, Endkonzentration: 1 mg/ml. Als Spaltungspuffer wird PBS-Puffer der Boehringer Mannheim GmbH Deutschland (Ident.-Nr. 2958686) eingesetzt. Pro Spaltungsansatz werden 2µg PSA-ACT in 40 µl Spaltungspuffer verwendet. Zur Spaltung der PSA-ACT-haltigen Proben werden die zu testenden aminhaltigen Nukleophile, die in Tabelle 1 aufgeführt sind, zur Probe dazugegeben und bei 25°C inkubiert. Nach der Inkubation werden die Proben über HPLC unter folgenden Bedingungen getrennt:
- Säule:: Reversed Phase Säule Poros R 1/H, 2.1 x 30 mm
- Elutionspuffer A:: 0.1% Trifluoressigsäure
- Elutionspuffer B:: 0.085% Trifluoressigsäure in Acetonitril / Wasser 70 / 30
- Gradient:: 20% B in A von 0 bis 2 min, linearer Anstieg bis 100% B innerhalb von 11 min; 100% B für 2 min.

Die Spaltungseffizienz der eingesetzten Substanzen ist in Tabelle 1 aufgeführt.

**Tabelle 1**

| Spaltungseffizienz der verschiedenen aminhaltigen Nukleophile bei 25°C | |
|---|---|
| **Aminhaltiges Nukleophil** | **% Spaltung nach 65h** |
| 0.1M Ethanolamin, pH 9.0 | 28% |
| 0.5M Ethanolamin, pH 9.0 | 37% |
| 1M Ethanolamin, pH 9.0 | 61% |
| 0.1M Diethanolamin, pH 9.0 | 17% |
| 0.1M Ethylendiamin, pH 9.0 | 8% |
| 1M TRIS, pH 9.0 | 14% |

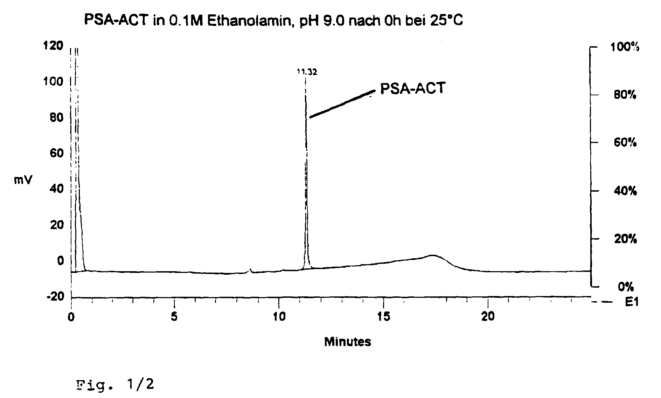

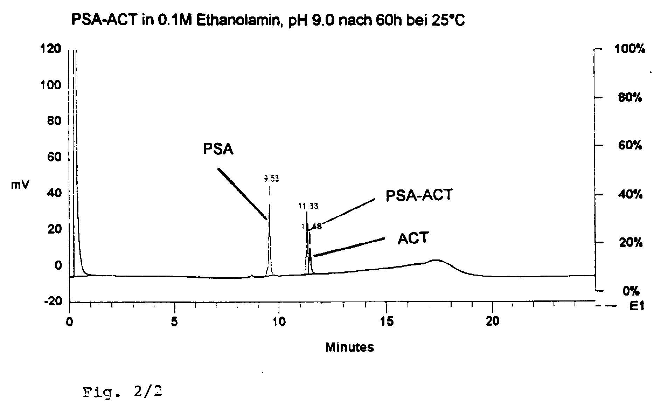

Es wird deutlich, daß mit Ethanolamin die größte Spaltungseffizienz erreicht werden kann. In den Figuren 1 und 2 sind beispielhaft HPLC-Chromatogramme der Spaltung von PSA-ACT aufgeführt.
Figur 1 zeigt ein HPLC-Chromatogramm von PSA-ACT in PBS-Puffer vor der Spaltung mit Ethanolamin.
Figur 2 zeigt ein HPLC-Chromatogramm von PSA-ACT nach der Spaltung in PSA und ACT mit Ethanolamin.

### Beispiel 2: Spaltung von PSA-ACT in malignen und benignen Seren mit 0,1 M Ethanolamin Endkonzentration

Die Durchführung des PSA_{gesamt}-Nachweises erfolgt entsprechend der in der Packungsbeilage zum Enzymun-Test® PSA der Boehringer Mannheim GmbH Deutschland (Ident. Nr. 1 555 332) angegebenen Vorgehensweise. Die Bestimmung der PSA_{frei}-Konzentration erfolgt wie in der Packungsbeilage zum Enzymun-Test® free PSA der Boehringer Mannheim GmbH Deutschland (Ident. Nr. 1 776 444) beschrieben. Die beiden Tests unterscheiden sich lediglich durch die Spezifität der eingesetzten Antikörper. Beim Nachweis von freiem PSA muß mindestens einer der Antikörper spezifisch für freies PSA sein. darf also komplexiertes PSA nicht binden, während beim Nachweis von PSA_{gesamt} sowohl freies als auch komplexiertes PSA erkannt werden muß. Der besseren Übersichtlichkeit halber werden jedoch in der Durchführungsvorschrift alle Antikörper als 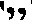 Anti-PSA-Antikörper" bezeichnet.

Das Testprinzip beider Verfahren ist ein 1 -Schritt-Sandwich-ELISA beruhend auf der Streptavidin-Technologie. Dabei wird die Probe zusammen mit einer Inkubationslösung, die biotinylierte Anti-PSA-Antikörper und mit Peroxidase (POD) markierte Anti-PSA-Antikörper enthält, in ein mit Streptavidin beschichtetes Kunststoffröhrchen gefüllt. Während der Inkubation bindet der biotinylierte Antikörper an die Festphase. Das in der Probe vorhandene PSA bindet an den biotinylierten Antikörper. Der mit POD markierte Antikörper bindet wiederum an das von biotinylierten Antikörper gebundene PSA. Die Detektion der an die Festphase gebundenen Sandwich-Komplexe erfolgt nach einem Wasch-Schritt über eine Indikatorreaktion. Dazu wird eine Substrat-Chromogen-Lösung, die ABTS® (2,2'-Azino-di-[3-ethyl-benzthiazolin-sulfonsäure (6)]-diammoniumsalz) und H₂O₂ enthält, zum Ansatz gegeben. Durch die enzymatische Aktivität der Peroxidase entwickelt sich in Abhängigkeit der Menge des gebundenen POD-markierten Antikörpers ein Farbstoff, der photometrisch nachgewiesen wird.

Bei einer Reihe von Human-Serum-Proben werden folgende Messungen durchgeführt: Zunächst wird in einem ersten Ansatz die PSA_{frei}-Konzentration nach dem Verfahren des Standes der Technik ohne jegliche Vorbehandlung der Probe bestimmt. Parallel dazu wird die PSA_{gesamt}-Konzentration auch nach dem Verfahren des Standes der Technik bestimmt. Das heißt, es werden zum Nachweis Antikörper eingesetzt, die PSA in freier und komplexierter Form binden. In einem dritten Ansatz wird die Probe erfindungsgemäß mit Ethanolamin versetzt (Endkonzentration 0,1 M) und nach 24-stündiger Inkubation bei 25°C die PSA_{frei}-Konzentration bestimmt. Die Ergebnisse für maligne und benigne Seren sind in Tabelle 2 dargestellt.

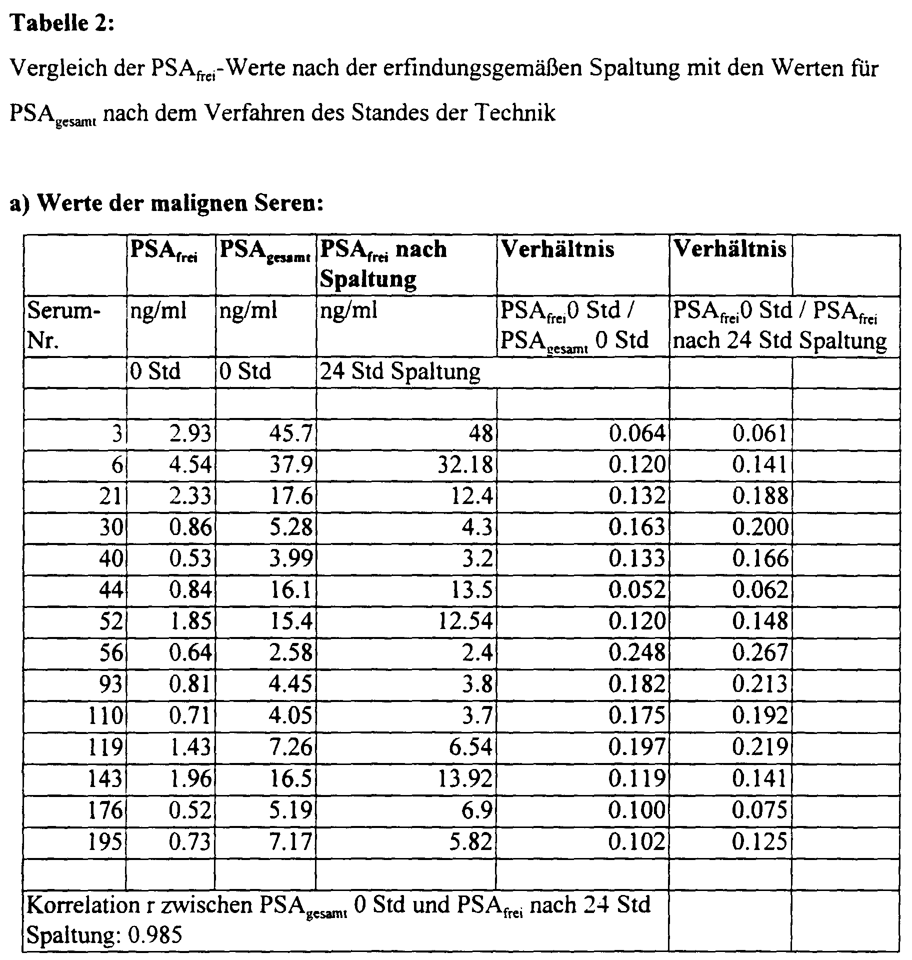

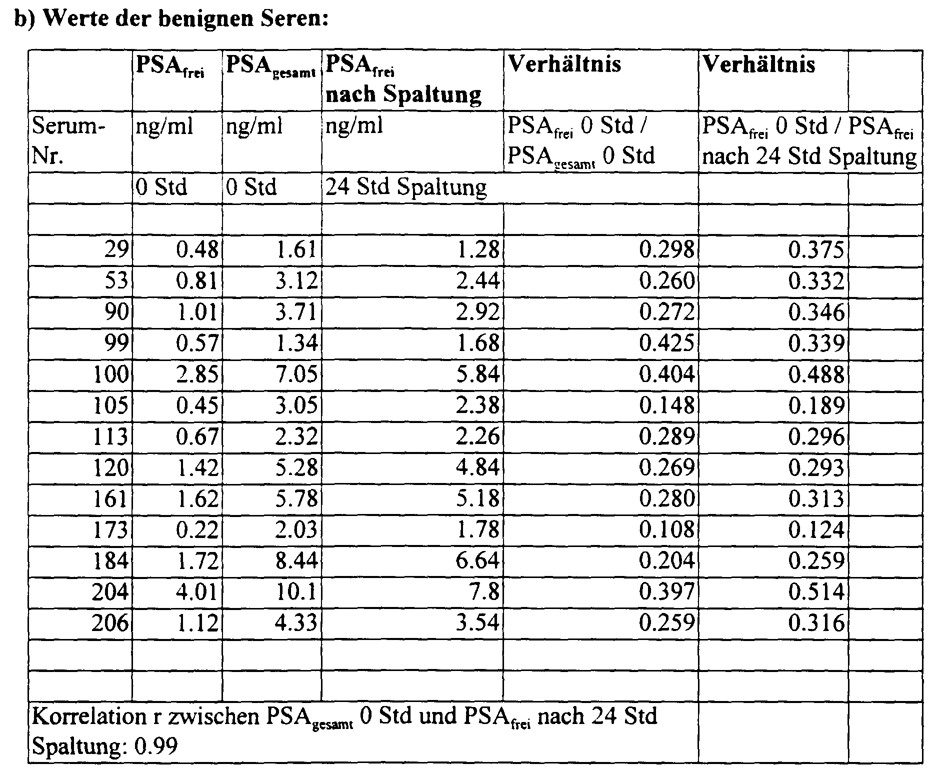

Es wird deutlich, daß die PSA_{gesamt}-Konzentration überraschenderweise der Konzentration an freiem PSA entspricht, die nach der erfindungsgemäßen Spaltung der PSA-Serpin-Komplexe ermittelt wird. Die gute Übereinstimmung der Quotienten PSA_{frei} 0 Std/PSA_{gesamt} nach 0 Std Spaltung und der PSA_{frei}0 Std/PSA_{frei} nach 24 Std Spaltung wird durch die Korrelationskoeffizienten von jeweils über 0,98 belegt. Damit ist gezeigt, daß mit Hilfe des erfindungsgemäßen Verfahrens eine Testführung mit einem Satz an Antikörpern, von denen einer spezifisch nur freies PSA bindet, ausreicht, um sowohl die PSA_{frei}- als auch die PSA_{gesamt}- und auch (durch rechnerisches Subtrahieren der PSA_{frei}-Konzentration von der PSA_{gesamt}-Konzentration) die PSA-Serpin-Konzentration zu bestimmen. Außerdem können in zuverlässiger Weise die für die Differenzierung zwischen malignen und benignen Prostata-Erkrankungen notwendigen aussagekräftigen Quotienten PSA_{frei}/PSA_{gesamt} ermittelt werden.

## Patentansprüche

1. Immunologisches Verfahren zur Bestimmung von PSA durch Inkubation der Probe mit mindestens einem Antikörper, der spezifisch an freies PSA, aber nicht an komplexiertes PSA bindet, dadurch gekennzeichnet, daß die Probe mit einem aminhaltigen Nukleophil versetzt wird.

2. Immunologisches Verfahren zur Bestimmung von freiem PSA, dem Gesamtgehalt an PSA und dem Gehalt an PSA-Serpin-Komplexen mit mindestens einem Antikörper, der spezifisch an freies PSA, aber nicht an komplexiertes PSA bindet, dadurch gekennzeichnet, daß vor der Bestimmung des Gesamtgehalts an PSA die Probe mit einem aminhaltigen Nukleophil versetzt wird.

3. Immunologisches Verfahren gemäß Anspruch 1 oder 2, dadurch gekennzeichnet, daß als aminhaltiges Nukleophil ein Aminoalkohol zugesetzt wird.

4. Immunologisches Verfahren gemäß Anspruch 3, dadurch gekennzeichnet, daß als aminhaltiges Nukleophil Ethanolamin zugesetzt wird.

5. Verwendung eines Aminoalkohols zur Herstellung von freiem PSA.

6. Verfahren zur Herstellung von freiem PSA aus komplexiertem PSA durch Inkubation einer Probe, die komplexiertes PSA enthält, mit einem Aminoalkohol.

7. Verwendung von freiem PSA, erhältlich durch ein Verfahren gemäß Anspruch 6 als Standard.

8. Verwendung von freiem PSA, erhältlich durch ein Verfahren gemäß Anspruch 6 für nicht-immunologische Untersuchungen.

9. Verwendung eines nukleophilen aminhaltigen Reagenz in einem immunologischen Verfahren zum Nachweis von PSA.

10. Reagenz, enthaltend ein nukleophiles aminhaltiges Reagens, zur Verwendung in einem Immunoassay zum Nachweis von PSA.
